# EUROPÄISCHE PATENTSCHRIFT

(11) **EP 2 263 997 B1**
(45) Veröffentlichungstag und Bekanntmachung des Hinweises auf die Patenterteilung: **14.09.2016**
(21) Anmeldenummer: 10005889.0
(22) Anmeldetag: 08.06.2010
(51) Int. Cl.: C07C 209/84, C07C 211/46

(54) **Verfahren zur Herstellung von aromatischen Aminen**
Method for manufacturing aromatic amines
Procédé destiné à la fabrication d'amines aromatiques

(30) Priorität: 18.06.2009 DE 102009025374
(43) Veröffentlichungstag der Anmeldung: 22.12.2010
(73) Patentinhaber: Covestro Deutschland AG, 51373 Leverkusen (DE)
(72) Erfinder: Sommer, Knut, Dr., 47804 Krefeld (DE); Lehner, Peter, Dr., 45481 Mülheim/Ruhr (DE); Lago, Andre, 22607 Hamburg (DE)
(74) Vertreter: Levpat

(56) Entgegenhaltungen:
- EP-A1- 2 028 176
- DATABASE CA [Online] CHEMICAL ABSTRACTS SERVICE, COLUMBUS, OHIO, US; 2007, IWAMOTO, KOJI ET AL: "Method for refining aniline" XP002599693 gefunden im STN Database accession no. 2007:828638 -& JP 2007 217405 A (SUMITOMO CHEMICAL CO) 30. August 2007 (2007-08-30)

## Beschreibung

Die Erfindung betrifft ein Verfahren zur Herstellung aromatischer Amine durch Hydrierung der entsprechenden aromatischen Nitroverbindungen und anschließende Reinigung. Unter aromatischen Aminen werden hier solche Verbindungen verstanden, die mindestens eine Aminogruppe an einem aromatischen Ring tragen. Letzterer kann beliebig substituiert bzw. mit anderen aromatischen Ringen kondensiert sein.

Die Reinigung dieser aromatischen Amine erfolgt dabei bevorzugt in der Weise, dass das jeweilige Amin zunächst mit einer *möglichst geringen Menge* einer *möglichst hoch konzentrierten* wässrigen Lösung einer Base vermischt wird, sodass es bevorzugt auf dieser Stufe noch nicht zur Ausbildung einer klar sichtbaren Phasentrennung kommt. *Erst im Anschluss daran* wird durch Zugabe eines Überschusses an Wasser eine Entmischung von organischer und wässriger Phase bewusst herbeigeführt. Die Lage der organischen Phase (oben oder unten) wird bevorzugt durch geeignete Wahl der physikalischen Randbedingungen *gezielt eingestellt.*

Die Herstellung der aromatischen Amine in dem erfindungsgemäßen Verfahren kann nach den aus dem Stand der Technik bekannten technischen Verfahren erfolgen. Ein solches Verfahren ist beispielsweise aus EP-A-1935871 für Toluylendiamin bekannt. Danach wird die Hydrierung der Nitroverbindungen bei Temperaturen von 100 bis 200 °C, bevorzugt von 120 bis 180 °C, besonders bevorzugt von 125 bis 170 °C und ganz besonders bevorzugt von 130 bis 160 °C in Gegenwart von Katalysatoren bei absoluten Drücken von 5 bis 100 bar, bevorzugt von 8 bis 50 bar und besonders bevorzugt von 10 bis 35 bar durchgeführt.

Als Nitroaromaten können im Prinzip alle technisch üblichen Nitroaromaten eingesetzt werden. Bevorzugt werden aromatische Mono- und / oder Diamine eingesetzt. Besonders bevorzugt werden Nitrobenzol, Nitrotoluol und Dinitrotoluol eingesetzt.

Als Reaktionsapparat kann beispielsweise der in WO-A-96/11052 beschriebene Schlammphasenreaktor eingesetzt werden. Geeignete andere Reaktoren sind z. B. in EP-A-0236 935 oder US-A-6350911 beschrieben. Selbstverständlich können auch mehrere gleiche oder Kombinationen unterschiedlicher geeigneter Reaktionsapparate eingesetzt werden.

Als Katalysatoren können alle zur katalytischen Hydrierung von aromatischen Nitroverbindungen bekannten Hydrierkatalysatoren eingesetzt werden. Gut geeignet sind insbesondere die Metalle der 8. Nebengruppe des Periodensystems der Elemente oder Mischungen derselben, die beispielsweise auf Trägermaterialien wie Kohlenstoff oder Oxiden von Magnesium, Aluminium und/oder Silizium aufgebracht sein können. Vorzugsweise werden Raney-Eisen, -Kobalt und/oder -Nickel, insbesondere Nickel-haltige Katalysatoren wie beispielsweise Raney-Nickel-Katalysatoren sowie Palladium oder Platin-haltige Katalysatoren auf Trägermaterialien eingesetzt, deren Herstellung und Anwendung als Hydrierkatalysator von aromatischen Nitroverbindungen wie z. B. Nitrobenzol, Nitrotoluolen, Dinitrotoluolen, chlorierten Nitroaromaten und anderen bekannt ist und bereits öfters beschrieben wurde (z. B. EP-A 0223035, EP-B-1066111, EP-A-1 512 459). Die dort beschriebenen Verfahren haben insbesondere für die Hydrierung von Dinitrotoluol Bedeutung.

Aromatische Amine sind wichtige Zwischenprodukte, welche preiswert und in großen Mengen zur Verfügung stehen müssen. Daher müssen beispielsweise für die Herstellung von Anilin Anlagen mit sehr großen Kapazitäten gebaut werden. Anilin ist beispielsweise ein wichtiges Zwischenprodukt bei der Herstellung von Methylendiphenyldiisocyanat (MDI) und wird im großtechnischen Maßstab in der Regel durch katalytische Hydrierung von Nitrobenzol hergestellt, wie es bspw. in DE-A-2201528, DE-A-3414714, US 3136818, EP-B-0 696 573, EP-B-0 696 574 und EP-A-1 882 681 beschrieben ist. Grundsätzlich kann auch im Falle von Anilin das Anilin aus allen technisch üblichen Verfahren zur Hydrierung von Nitrobenzol stammen. Bevorzugt wird die Hydrierung von Nitrobenzol in der Gasphase an ortsfesten, heterogenen Trägerkatalysatoren, wie z. B. Pd auf Aluminiumoxid oder Kohleträgern, in Festbettreaktoren bei einem absoluten Druck von 2 - 50 bar und einer Temperatur im Bereich von 250 - 500 °C unter adiabatischen Bedingungen in Kreisgasfahrweise, d. h. unter Rückführung von während der Hydrierung nicht umgesetztem Wasserstoff, durchgeführt (siehe EP-A-0696573 und EP-A- 0696574).

Bei der Herstellung aromatischer Amine werden neben den Zielprodukten auch Wasser und organische Nebenkomponenten gebildet. Diese organischen Nebenkomponenten müssen vor einer weiteren Verwendung der aromatischen Amine abgetrennt werden. Besonders problematisch ist die Abtrennung von solchen Nebenkomponenten, deren Siedepunkte dem des herzustellenden Amins sehr ähnlich sind, weil hier der destillative Aufwand erheblich ist. Im Fall der Herstellung von Anilin (Sdp. = 184 °C) stellt insbesondere die Abtrennung von Phenol (Sdp. = 182 °C) eine große Herausforderung für die Destillationstechnik dar, was sich in der Verwendung langer Destillationskolonnen mit großer Trennstufenzahl und hohen Rücklaufverhältnissen mit entsprechend hohem Investitions- und Energieaufwand widerspiegelt. Verbindungen mit phenolischen Hydroxygruppen, d. h. Verbindungen, die mindestens eine Hydroxygruppe an einem aromatischen Ring tragen, können generell bei der Aufarbeitung aromatischer Amine problematisch sein. Im Fall des Anilins sind hier neben dem bereits erwähnten Phenol insbesondere die verschiedenen Aminophenole zu nennen.
Die Reinigung aromatischer Amine ist daher nicht trivial und besitzt große industrielle Bedeutung. Neuere Ansätze widmen sich insbesondere der erwähnten Problematik im Zusammenhang mit Verbindungen mit phenolischen Hydroxygruppen. Der Lösungsansatz besteht darin, die Verbindungen mit phenolischen Hydroxygruppen durch Reaktion mit geeigneten Basen in die entsprechenden Salze zu überführen, welche sich als nichtflüchtige Verbindungen wesentlich leichter abtrennen lassen.

So wird in JP-A-49-035341 ein Verfahren beschrieben, bei dem das zu reinigende Amin, dort Anilin, mit festen Alkalihydroxiden in einem Festbett in Kontakt gebracht und erst anschließend in die Destillation geleitet wird, bzw. bei dem die Destillation in Gegenwart des festen Alkalihydroxides in Anteilen von 0,1 - 3 Massen-%, bezogen auf die zu destillierende Anilinmenge, durchgeführt wird. Hierdurch wird die Abtrennung kritischer Komponenten wie der Aminophenole vereinfacht. Nachteilig bei diesem Verfahren ist jedoch der Einsatz von hohen molaren Überschüssen der festen Alkalihydroxide in Bezug auf die zu entfernenden sauren Nebenkomponenten bzw. die Unmöglichkeit der genauen Dosierung der alkalischen Verbindungen. Dies kann einerseits bei Überdosierung zu Korrosionsproblemen, Ausfällungen und hochviskosen Sumpfphasen in der Destillationskolonne und andererseits bei Unterdosierung zu einer unvollständigen Entfernung der kritischen Komponenten führen.

US-A-2005 080294 beschreibt ein Verfahren zur Abtrennung von Verbindungen mit phenolischen Hydroxygruppen (dort "phenolic compounds" genannt) von aromatischen Aminen, bei dem dem zu reinigenden Amin vor der Destillation eine Base im molaren Verhältnis von 1 : 1 bis zu 4 : 1 bezogen auf die "phenolic compounds" zugesetzt wird, optional in Gegenwart von Polyolen. US-A-2005 080294 lehrt nicht im Einzelnen, was mit den Salzen, die bei Reaktion der "phenolic compounds" mit den Basen entstehen, geschieht. In Beispiel 6 wird lediglich erwähnt, dass überschüssiges festes KOH durch Zusatz von Polyethylenglykol (PEG) in Lösung gebracht wird. Welche Konsequenzen damit verbunden sind, ist US-A-2005 080294 nicht zu entnehmen. Auf die Salze der "phenolic compounds" selbst geht US-A-2005 080294 gar nicht ein.

Salze jedoch, und zwar nicht nur überschüssige Base, sondern auch die Salze der Verbindungen mit phenolischen Hydroxygruppen, sind im Allgemeinen in aromatischen Aminen nur wenig löslich, sodass die große Gefahr besteht, dass sie sich in der Destillationskolonne, im Sumpf der Destillationskolonne und/oder im Verdampfer der Destillation über die Löslichkeitsgrenze hinweg anreichern und dann ausfallen. Solche Feststoffniederschläge können den Destillationsprozess empfindlich stören, sodass eine Unterbrechung des Destillationsvorgangs erforderlich wird, was in der großtechnischen Produktion, die kontinuierlich betrieben wird, zu erheblichen Schwierigkeiten und sogar zu Produktionsausfällen führen kann. US-A-2005 080294 befasst sich mit dem Problem der Zuverlässigkeit und Standzeit des Verfahrens jedoch nicht. Der Fachmann erfährt aus US-A-2005 080294 auch nicht, dass die Anwesenheit der während der Reaktion der Verbindungen mit phenolischen Hydroxygruppen mit den Basen entstehenden Salze zu Feststoffabscheidung, Fouling und/oder starkem Viskositätsanstieg während der Destillation führen kann. Auf Details der Destillationstechnik geht US-A-2005 080294 gar nicht ein. Der Fachmann erfährt aus USA-2005 080294 daher nicht, wie er diese mit hoher Wahrscheinlichkeit auftretenden Probleme lösen soll. USA-2005 080294 lehrt nur die optionale zusätzliche Zugabe von PEG, um überschüssiges festes KOH in Lösung zu bringen. Eine solche Zugabe von PEG in die Destillation ist aufgrund der hohen Kapazitäten bei der Herstellung aromatischer Amine (insbesondere Anilin) jedoch wirtschaftlich nachteilig.

EP-A-1 845 079 beschreibt ein Verfahren zur Reinigung von Anilin durch Zugabe einer wässrigen Alkalimetallhydroxid-Lösung vor oder während der Destillation, wobei die Probleme durch Feststoffabscheidung, Fouling und/oder starken Viskositätsanstieg bei der Destillation dadurch verhindert werden, dass die Sumpfphase der Destillation partiell ausgeschleust, mit Wasser oder verdünnter Alkalihydroxidlösung gewaschen und die organische gewaschene Phase wieder in die Destillation zurückgeführt wird. Nachteilig ist hier die Notwendigkeit eines zusätzlichen Verfahrensschrittes zur Aufrechterhaltung eines zuverlässigen Betriebs.

EP-A-2 028 176 beschreibt ein Verfahren zur Reinigung von aromatischen Aminen, bei dem das nach Abtrennung des Prozesswassers erhaltene rohe Amin mit wässriger Alkalimetallhydroxid-Lösung versetzt und das so erhaltene Verfahrensprodukt destilliert wird. Der Sumpf der Destillationskolonne wird teilweise bis vollständig ausgeschleust und teilweise über zwei seriell oder parallel geschaltete Verdampfer (E¹) und (E²) verdampft. Hierdurch soll mit minimalem apparativem und energetischem Aufwand eine maximale Abreicherung des wertvollen Amins im Sumpf der Destillationskolonne erreicht werden.

Bei allen bisher genannten Verfahren wird das aromatische Amin *in Gegenwart einer Base* destilliert. Bei dieser Verfahrensweise müssen Probleme durch Feststoffabscheidung, Fouling und/oder starken Viskositätsanstieg bei der Destillation durch aufwändige und/oder teure Maßnahmen verhindert werden.

Als Alternative zur Entfernung von Verbindungen mit phenolischen Hydroxygruppen aus Anilin während der Destillation beschreibt JP-A-08-295654 eine Extraktion mit verdünnter wässriger Alkalihydroxidlösung. In einer bevorzugten Ausführungsform, welche allein in den Beispielen 1 bis 5 beschrieben wird, erfolgt dies in der Weise, dass die nach Vermischen des phenolhaltigen Anilins mit der Alkalihydroxidlösung erhaltene wässrige Phase das Alkalihydroxid in einer Konzentration von 0,1 bis 0,7 Massen-% enthält und die nach Phasentrennung erhaltene organische Phase destilliert wird. Das Phenol wird im Extraktionsschritt als Alkaliphenolat größtenteils in die wässrige Phase transferiert und so bei der Phasentrennung vom zu reinigenden Anilin getrennt. In den Beispielen 1 bis 5 wird das Produkt der Nitrobenzolhydrierung direkt, also ohne vorherige Abtrennung von Prozesswasser, mit wässriger Natriumhydroxidlösung behandelt, und das molare Verhältnis von NaOH zu Phenol beträgt mindestens 69 : 1 (Beispiel 2). Die Beschränkung auf die geringe Konzentration von ≤ 0,7 Massen-% soll eine Verbesserung der Phasentrennung bewirken. Nachteilig bei diesem Verfahren sind der hohe NaOH-Verbrauch und das Anfallen sehr großer Mengen alkaliphenolathaltigen Abwassers infolge der geringen Konzentration der Alkalihydroxid-Lösungen.

EP-A-1 845 080 beschreibt ein Verfahren zur Reinigung von Anilin durch Extraktion mit wässriger Alkalimetallhydroxid-Lösung einer Konzentration > 0,7 Massen-%, wobei Konzentration und Temperatur so eingestellt werden, dass die wässrige Phase bei der anschließenden Phasentrennung immer die untere Phase darstellt. Hierdurch wird ein stabiler Betrieb gewährleistet, weil keine Probleme durch Phasenumkehr auftreten. Auch in diesem Verfahren wird nur mit relativ gering konzentrierten Alkalimetallhydroxidlösungen (in den Beispielen zwischen 0,8 und 2,5 Massen% bezogen auf die Masse der Alkalimetallhydroxidlösung) und einem hohen molaren Überschuss an Alkalimetallhydroxid (in den Beispielen minimal 12,52 : 1) gearbeitet. Das Massenverhältnis von organischem zu wässrigem Anteil bei der Extraktion beträgt in den Beispielen nur 5,0 oder weniger.

JP-A-2007217405 beschreibt ein Verfahren, in dem das phenolhaltige Anilin *mindestens zweimal* mit wässriger Alkalimetallhydroxidlösung so in Kontakt gebracht wird, dass die Konzentration an Alkalimetallhydroxid in der wässrigen Phase zwischen 0,1 und 0,7 Massen-% liegt. Dies wird erreicht, indem zu dem nach Phasentrennung erhaltenen Rohanilin zunächst Wasser *und danach* eine relativ konzentrierte (25%ig) wässrige NaOH-Lösung gegeben wird (siehe Beispiele). Die in den Beispielen jeweils in Summe über beide Extraktionsschritte verwendeten molaren Überschüsse an Alkalimetallhydroxid in Bezug auf Phenol betragen mindestens 10,6. Dies kann Beispiel 3 entnommen werden: Dort werden 174 g eines Rohanilins mit einem Phenolgehalt von 522 ppm gereinigt (entsprechend 0,966 mmol Phenol), und es erfolgt eine 2-malige Zugabe von jeweils 0,82 g einer 25%igen NaOH-Lösung (in Summe entsprechend 10,25 mmol NaOH). Es wird also auch in diesem Verfahren ein relativ großer molarer Überschuss an Alkalimetallhydroxid benötigt, mit all den bereits bei JP-A-08-295654 erwähnten Nachteilen.

Bei allen erwähnten Verfahren zur Reinigung von aromatischen Aminen mit Hilfe einer extraktiven Basebehandlung kommt aus wirtschaftlichen Gründen in der großtechnischen Produktion als Base nur eine auch in großen Mengen preisgünstig verfügbare in Frage; in der Praxis fällt daher die Wahl fast immer auf wässrige Lösungen von Alkalimetallhydroxiden, insbesondere von Natriumhydroxid. Nach dem Stand der Technik wird die Base entweder in Form einer relativ stark verdünnten wässrigen Lösung zugegeben oder das zu reinigende Amin wird vor der Basezugabe mit Wasser versetzt bzw. Prozesswasser nicht abgetrennt, sodass die Konzentration an Base im wässrigen Anteil des so erhaltenen Verfahrensproduktes nur gering und das Massenverhältnis von organischem zu wässrigem Anteil relativ klein ist. Bspw. beträgt das Massenverhältnis von organischem zu wässrigem Anteil im ersten Extraktionsschritt von Beispiel 3 in JP-A-2007217405 nur 2,6 (174 g Rohanilin zu 67 g Wasser und 0,82 g NaOH-Lösung), wobei in dieser Rechnung noch nicht einmal berücksichtigt ist, dass das eingesetzte rohe Anilin mehrere Prozent Wasser enthalten kann, sodass der tatsächliche Wert wahrscheinlich noch kleiner ist. Diese Vorgehensweise hat infolge der schlechten Mischbarkeit des wässrigen, die Base enthaltenden Anteils im so erhaltenen Verfahrensprodukt mit dem rohen aromatischen Amin zur Folge, dass die Reaktion von sauren Verunreinigungen mit Baseteilchen nur an der Phasengrenzfläche stattfindet; daher müssen sehr große molare Überschüsse an Base eingesetzt werden.

Unter Reaktivitätsgesichtspunkten ideal wäre eine organische Base, die mit dem aromatischen Amin gut mischbar ist. Diese könnte in stöchiometrischer Menge eingesetzt und das gebildete Salz anschließend leicht abgetrennt werden. Da in der großtechnischen Produktion dieser Weg aus wirtschaftlichen Gründen jedoch nicht tragbar ist, muss nach anderen Lösungen für die geschilderte Problematik gesucht werden.

Die Aufgabe der vorliegenden Erfindung war es daher, ein Verfahren zur Herstellung und Reinigung von aromatischen Aminen bereitzustellen, welches die Abtrennung von Verbindungen mit phenolischen Hydroxygruppen durch eine Behandlung mit Base(n) so ermöglicht, dass
- mit möglichst geringen molaren Überschüssen der eingesetzten Base in Bezug auf die zu entfernenden sauren Verbindungen gearbeitet werden kann,
- ein stabiler Betrieb ohne die Gefahr einer Phasenumkehr bei der Trennung organischer und wässriger Phasen gewährleistet ist,
- Probleme wie Feststoffabscheidung, Fouling und/oder starker Viskositätsanstieg während der Destillation vermieden werden.

Die Erfindung betrifft ein Verfahren zur Herstellung von aromatischen Aminen, bei dem
a) die aromatischen Amine durch Hydrierung der entsprechenden aromatischen Nitroverbindungen in Gegenwart eines Katalysators hergestellt werden und das bei der Hydrierung gebildete Wasser (Prozesswasser) durch Phasentrennung abgetrennt wird, wobei die rohen aromatischen Amine erhalten werden, und
b) die aus Schritt a) erhaltenen rohen aromatischen Amine anschließend gereinigt werden, indem
   b) (i) die aus Schritt a) erhaltenen rohen aromatischen Amine mit mindestens einer wässrigen Lösung einer Base vermischt werden, wobei die eingesetzte Menge und die Basen-Konzentration der wässrigen Lösung der Base so gewählt werden, dass das molare Verhältnis der Summe aller Basen, die in Verfahrensschritt b) zu den aromatischen Aminen zugegeben werden, zu den phenolischen Hydroxygruppen, die in den aus Schritt a) erhaltenen aromatischen Aminen enthalten sind, nicht größer als 10, bevorzugt zwischen 1 und 10 ist, und dass das Massenverhältnis von organischen Bestandteilen zu Wasser größer als 10 ist, und
   b) (ii) das aus Schritt b) (i) erhaltene Gemisch mit Wasser vermischt wird, wobei das Massenverhältnis des aus Schritt b) (i) erhaltenen Gemisches zu dem in Schritt b) (ii) zugegebenen Wasser im Bereich zwischen 0,05 und 20, bevorzugt im Bereich zwischen 0,1 und 10, besonders bevorzugt zwischen 0,2 und 5, liegt, und
   b) (iii) das aus Schritt b) (ii) erhaltene zweiphasige Gemisch anschließend in eine die aromatischen Amine enthaltende organische Phase und eine wässrige Phase aufgetrennt wird.

Bevorzugt werden dabei Temperatur und Druck bei der Phasentrennung in Schritt b) (iii) so gewählt, dass die organische Phase bei der Phasentrennung eine zuvor festgelegte definierte Lage einnimmt (entweder oben oder unten).

Das in Schritt b) (i) geforderte Massenverhältnis von organischen Bestandteilen zu Wasser von größer als 10 bevorzugt größer als 50, besonders bevorzugt größer als 100 und ganz besonders bevorzugt größer als 150 führt dazu, dass insgesamt mit geringeren molaren Überschüssen an Base gearbeitet werden kann als in der Technik bei extraktiven Reinigungsverfahren bisher üblich. Üblicherweise wird dabei das Massenverhältnis einen Wert von 10000 nicht übersteigen.

Der Grund hierfür liegt darin, dass durch den gegenüber dem Stand der Technik deutlich verringerten Anteil an wässriger, d. h. mit dem aromatischen Amin nur schwer mischbarer, Phase die Reaktion zwischen den sauren Verunreinigungen und den Baseteilchen erheblich erleichtert wird. Bevorzugt findet auf dieser Stufe des Verfahrens keine klar sichtbare Phasentrennung statt, wenn das Rühren unterbrochen wird. Erst nach Abschluss der eigentlichen Säure-Base-Reaktion wird in Schritt b) (ii) eine signifikante Menge an Wasser zugegeben, und in der anschließenden Phasentrennung in Schritt b) (iii) werden Wasser, darin gelöste Salze und überschüssige Base abgetrennt.

Die in Schritt b) (i) eingesetzten und aus Schritt a) erhaltenen rohen aromatischen Amine können aus allen bekannten technischen Verfahren zur Herstellung von aromatischen Aminen stammen. Insbesondere geeignet sind aromatische Amine, die durch katalytische Reduktion der entsprechenden Nitroverbindungen erhalten wurden. Derartige Verfahren sind oben für Toluylendiamin und Anilin beispielhaft ausgeführt. Das hierbei anfallende Prozesswasser wird durch dem Fachmann bekannte Phasentrennung vom organischen Reaktionsprodukt abgetrennt. Das auf diese Weise gewonnene rohe Amin, das in Schritt a) erhalten wird, enthält bevorzugt noch einen der Löslichkeit von Wasser im rohen Amin unter den gegebenen physikalischen Randbedingungen (insbesondere Temperatur und Druck) entsprechenden Restwassergehalt, der zwischen 0,01 und 20 Massen-%, bezogen auf die Masse des rohen Amins (gleichbedeutend mit dem in Schritt a) erhaltenen aromatischen Amin), betragen kann.

Ob bei der Phasentrennung in Schritt b) (iii) die organische Phase die obere oder die untere ausbildet, ist für das Funktionieren des Verfahrens unerheblich. Abhängig von den apparativen Gegebenheiten können beide Zustände von Vorteil sein. Ist es unter den gegebenen Voraussetzungen einer Produktionsanlage bspw. erwünscht, dass die organische Phase bei der Phasentrennung die untere Phase ist, werden die physikalischen Randbedingungen, insbesondere die Temperatur, entsprechend eingestellt. Wichtig ist nur, dass nicht während des Prozesses unerwartet eine Phasenumkehr auftritt. Dies ist mit dem erfindungsgemäßen Verfahren auf einfache Weise vermeidbar.

Abhängig davon, auf welches Niveau der Gehalt an Verbindungen mit phenolischen Hydroxygruppen gesenkt werden soll, kann die aus Schritt b) (iii) erhaltene, die aromatischen Amine enthaltende organische Phase einer weiteren Basen-Behandlung unterworfen werden, d. h. der Verfahrensschritt b) wird um weitere Reinigungsstufen erweitert.

Dazu wird
b) (iv) die aus Schritt b) (iii) erhaltene die aromatischen Amine enthaltende organische Phase mit mindestens einer wässrigen Lösung einer Base vermischt, wobei die eingesetzte Menge und die Basen-Konzentration der wässrigen Lösung der Base so gewählt werden, dass das molare Verhältnis der Summe aller Basen, die in Verfahrensschritt b) zu den aromatischen Aminen zugegeben werden, zu den phenolischen Hydroxygruppen, die in den aus Schritt a) erhaltenen aromatischen Aminen enthalten sind, nicht größer als 10, bevorzugt zwischen 1 und 10 ist, und dass das Massenverhältnis von organischen Bestandteilen zu Wasser größer als 10 ist, und
b) (v) das aus Schritt b) (iv) erhaltene Gemisch mit Wasser vermischt wird, wobei das Massenverhältnis des aus Schritt b) (iv) erhaltenen Gemisches zu dem in Schritt b) (v) zugegebenen Wasser im Bereich zwischen 0,05 und 20, bevorzugt im Bereich zwischen 0,1 und 10, besonders bevorzugt im Bereich zwischen 0,2 und 5, liegt, und
b) (vi) das aus Schritt b) (v) erhaltene zweiphasige Gemisch anschließend in eine die aromatischen Amine enthaltende organische Phase und eine wässrige Phase aufgetrennt wird.

Bevorzugt werden dabei Temperatur und Druck bei der Phasentrennung in Schritt b) (vi) so gewählt, dass die organische Phase bei der Phasentrennung eine zuvor festgelegte definierte Lage einnimmt (entweder oben oder unten).

Prinzipiell können noch weitere Basenbehandlungen angeschlossen werden; im Allgemeinen ist dies jedoch nicht erforderlich.

Erforderlichenfalls kann die nach der Phasentrennung in Schritt b) (iii) bzw. b) (vi) erhaltene die aromatischen Amine enthaltende organische Phase mit Wasser in einem ein- oder mehrstufigen Prozess gewaschen werden, um noch die letzten Reste an überschüssiger Base und Salzen der Verbindungen mit phenolischen Hydroxygruppen zu entfernen.

In jedem der beschriebenen Verfahrensschritte b) (ii) und b) (v) kann das eingesetzte Wasser aus einer beliebigen Quelle stammen. So sind vollentsalztes Wasser, Betriebswasser oder Prozesswasser gleichermaßen geeignet. Aus wirtschaftlichen Gründen wird bevorzugt Betriebswasser, besonders bevorzugt Prozesswasser eingesetzt. Mit Prozesswasser ist dasjenige Wasser gemeint, welches bei der Herstellung der aromatischen Amine in Schritt a) gebildet wird. Dieses kann entweder so eingesetzt werden, wie es nach der Phasentrennung anfällt, oder zuvor noch durch Destillation oder andere Maßnahmen gereinigt werden.

Als Base werden bevorzugt Alkalimetallhydroxide verwendet, besonders bevorzugt Natriumhydroxid oder Kaliumhydroxid. Auch der Einsatz von anderen wasserlöslichen basischen Verbindungen ist grundsätzlich denkbar. Konzentration und Menge der zugegebenen wässrigen Lösung der Base werden so gewählt, dass die geforderten Parameter in Schritt b) (i) bzw. b) (iv) bzgl. des Massenverhältnisses von organischem zu wässrigem Anteil und bzgl. des molaren Überschusses an Base eingehalten werden. Zu diesem Zweck muss die Konzentration der Verbindungen mit phenolischen Hydroxygruppen im zu reinigendem Amin bestimmt werden. Dies geschieht bevorzugt durch gängige analytische Methoden, besonders bevorzugt durch Gaschromatographie. Die eingesetzte Lösung der Base sollte bevorzugt möglichst hoch konzentriert sein, wobei die maximal einsetzbare Konzentration nur durch die Löslichkeitsgrenze der Base in Wasser unter den gegebenen Bedingungen limitiert wird. Bevorzugt wird eine Lösung von Natriumhydroxid in Wasser eingesetzt, wobei der Massenanteil an Natriumhydroxid bevorzugt mindestens 10 %, besonders bevorzugt mindestens 20 % und ganz besonders bevorzugt mindestens 30 %, bezogen auf die Gesamtmasse der Natriumhydroxidlösung, beträgt. Außerordentlich besonders bevorzugt wird kommerziell erhältliche 32%ige Natronlauge eingesetzt.

Die Temperatur bei Schritt b) (i) bzw. b) (iv) liegt je nach Konzentration der wässrigen Baselösung bevorzugt im Bereich zwischen 20 °C und 160 °C, besonders bevorzugt zwischen 30 °C und 100 °C, ganz besonders bevorzugt zwischen 50 °C und 95 °C. Bevorzugt liegt die Temperatur während Schritt b) (ii) und b) (iii) bzw. b) (v) und b) (vi) und ggf. der abschließenden Wäsche in den gleichen Bereichen.

Die Wahl der geeigneten Kombination der Konzentration der wässrigen Baselösung und der Temperatur während der Extraktion richtet sich neben der Erreichung einer definierten Lage der organischen Phase bei der Phasentrennung nach den jeweils relevanten verfahrenstechnischen und ökonomischen Kriterien. So kann einerseits zur Begrenzung der Wasserlöslichkeit des aromatischen Amins eine Minimierung der Temperatur sinnvoll sein, andererseits kann es verfahrenstechnisch von Vorteil sein, das rohe Amin bei höherer Temperatur nach der Reaktion zu kondensieren und bei gleicher Temperatur dann auch zu extrahieren. Auch kann es sinnvoll sein, die Effektivität der Durchmischung in Schritt b) (i) bzw. b) (iv) durch Erhöhung der Temperatur zu steigern.

Für die Einmischung der wässrigen Base in das aromatische Amin (Schritte b) (i) und b) (iv)) können alle dem Fachmann bekannten Methoden und Apparaturen zur Mischung von flüssigen Phasen eingesetzt werden, wie z. B. statische Mischer, Düsen, Mischpumpen oder Rührer. Für die extraktive Wäsche (Schritte b) (ii) und b) (iii) bzw. b) (v) und b) (vi)) und für ggf. weitere notwendige Waschstufen können alle dem Fachmann bekannten Methoden und Apparaturen zur Extraktion, wie z. B. Mixer-Settler oder Extraktionskolonnen eingesetzt werden. Die extraktive Basebehandlung kann im Gleich- oder Gegenstrom erfolgen. In einer bevorzugten Ausführungsform wird eine zweistufige Mixer-Settler-Apparatur im Gegenstrom für die extraktive Basebehandlung eingesetzt. Zur Absenkung der notwendigen Trenn- und Verweilzeiten können die Abscheider mit Koaleszierhilfen wie z. B. Gestricken, Platten oder Füllkörpern versehen werden.

Alle weiter oben beschriebenen Verfahren zur Reinigung von aromatischen Aminen, bei denen die Base während einer Destillation im rohen Amin vorhanden ist, leiden unter Problemen wie Feststoffabscheidung, Fouling und/oder starkem Viskositätsanstieg während der Destillation. Auch im erfindungsgemäßen Verfahren können die aromatischen Amine erforderlichenfalls zusätzlich zur extraktiven Basebehandlung durch Destillation noch weiter gereinigt werden. Diese Destillation kann entweder vor oder nach der Basebehandlung (bzw. ggf. nach der abschließenden Wäsche) durchgeführt werden. Die genannten Phänomene von Feststoffabscheidung, Fouling und/oder starkem Viskositätsanstieg stellen jedoch im erfindungsgemäßen Verfahren keine Schwierigkeiten dar, denn überschüssige Base und die Salze der Verbindungen mit phenolischen Hydroxygruppen, welche ursächlich für diese Probleme sind, sind im erfindungsgemäßen Verfahren *während* der Destillation nicht vorhanden, entweder weil die Destillation vor der Basebehandlung durchgeführt wird oder weil die genannten anorganischen Bestandteile in den Schritten b) (iii) bzw. b) (vi) und/oder bei der ggf. abschließenden Wäsche abgetrennt werden.

Die nach- oder vorgeschalteten Destillationsschritte können in allen dem Fachmann geläufigen Varianten ausgestaltet und unter verschiedensten Bedingungen betrieben werden. So kann eine Destillation z. B. in einer oder mehreren Glockenboden- oder Packungskolonnen, aber auch in Trennwandkolonnen erfolgen. Dabei können Leichtsieder und Schwersiederabtrennung in verschiedenen Kolonnen, aber auch gemeinsam in einer Kolonne unter Seitenstromentnahme des aromatischen Amins erfolgen.

Das erfindungsgemäße Verfahren ist grundsätzlich auf alle aromatischen Amine anwendbar. Das zu reinigende Amin kann dabei aus allen technisch üblichen Verfahren zur Herstellung von aromatischen Aminen stammen. Besonders geeignet ist das erfindungsgemäße Verfahren für die Reinigung von Anilin. Das so gereinigte Anilin kann anschließend nach den aus dem Stand der Technik bekannten Verfahren mit Formaldehyd in Gegenwart eines sauren Katalysators zu Di- und Polyaminen der Diphenylmethanreihe umgesetzt werden. Die Di- und Polyamine können anschließend nach den aus dem Stand der Technik bekannten Verfahren, bevorzugt mit Phosgen, zu den entsprechenden Di- und Polyisocyanaten der Diphenylmethanreihe umgesetzt werden.

Die erfolgreiche Durchführung der extraktiven Basebehandlung eines aromatischen Amins mit so großen Verhältnissen von organischem zu wässrigem Anteil wie in dem erfindungsgemäßen Verfahren gefordert ist nach dem Stand der Technik überraschend, da bisher angenommen wurde, dies würde zu einer verminderten Extraktionseffizienz und verlängerten Trennzeiten führen (vgl. bspw. EP-A-1 845 080, [0013], Z. 15 - 17).

### Beispiele

Die nachfolgend beschriebenen Beispiele wurden in einem doppelwandigen Scheidetrichter mit KPG-Rührer durchgeführt, wobei zur Thermostatisierung 30 °C warmes Wasser durch den Hohlraum zwischen den beiden Glaswänden geleitet wurde.

Gereinigt wurde ein rohes Anilin mit einem Wassergehalt von 1500 ppm (bestimmt nach Karl Fischer) und einem Phenolgehalt (bestimmt durch Gaschromatographie) von 998 ppm, das aus der Hydrierung von Nitrobenzol an einem Pd/V/Pb-Katalysator auf Aluminiumoxid (analog EP 1 882 681 A1, Beispiel 3) erhalten wurde.

Die nach den Phasentrennungen jeweils erhaltenen organischen Phasen wurden lediglich aus Gründen der Vereinfachung des Arbeitsablaufs im Laboratorium aufgeteilt und nur teilweise dem nächsten Verarbeitungsschritt zugeführt,- *die zuzugebenden Mengen an NaOH-Lösung und Wasser wurden der verringerten Menge jeweils angepasst.* In einer großtechnischen Anwendung würden diese organischen Phasen bevorzugt vollständig der nächsten Verarbeitungsstufe zugeführt.

### Beispiel 1 (erfindungsgemäß, geringe Mengen an Wasser nach Basebehandlung zugegeben)

1000 g des rohen Anilins (Phenolgehalt 998 ppm) wurden mit 5,00 g 32%iger NaOH-Lösung (d. h. das Massenverhältnis von organischen Bestandteilen zu Wasser beträgt 154) versetzt, und die erhaltene Mischung wurde 3 Minuten lang gründlich verrührt (Schritt b) (i)). Sodann wurden 250 g Wasser zugegeben, und die erhaltene Mischung wurde 3 Minuten lang gründlich verrührt (Schritt b) (ii)). Man ließ stehen, bis sich die Phasen trennten, wobei die organische Phase die untere Phase ausbildete und es keinerlei Probleme mit zeitweiliger Phasenumkehr gab. Nach Phasentrennung (Schritt b) (iii)) wurden der Phenolgehalt (durch Gaschromatographie) und der Natriumgehalt (durch Atomabsorptionsspektroskopie) der organischen Phase bestimmt. 800 g der organischen (nun ca. 4,6 % Wasser enthaltenden) Phase wurden in einem nächsten Schritt mit 4,00 g 32%iger NaOH-Lösung versetzt, und die erhaltene Mischung wurde 3 Minuten lang gründlich verrührt (Schritt b) (iv)). Sodann wurden 200 g Wasser zugegeben, und die erhaltene Mischung wurde 3 Minuten lang gründlich verrührt (Schritt b) (v)). Man ließ stehen, bis sich die Phasen trennten, wobei die organische Phase die untere Phase ausbildete und es keinerlei Probleme mit zeitweiliger Phasenumkehr gab. Nach der Phasentrennung (Schritt b) (vi)) wurde die organische Phase wie beschrieben analysiert. 600 g der nach der zweiten Phasentrennung (Schritt b) (vi)) erhaltenen organischen Phase wurden schließlich noch mit 160 g Wasser 3 Minuten lang gründlich verrührt *(Wäsche),* und die nach Phasentrennung erhaltene organische Phase wurde wie beschrieben analysiert.

### Beispiel 2 (erfindungsemäß, große Mengen an Wasser nach Basebehandlung zugegeben)

Die prinzipielle Durchführung erfolgte wie in Beispiel 1 beschrieben, jedoch wurden in Schritt b) (ii) 1000 g (anstatt 250 g) Wasser und in Schritt b) (v) 800 g (anstatt 200 g) Wasser zugegeben. In der abschließenden Wäsche wurden 640 g (anstatt 160 g) Wasser eingesetzt. Es wurden wiederum keinerlei Probleme mit zeitweiliger Phasenumkehr beobachtet; die organische Phase war bei Phasentrennungen stets die untere Phase. Die nach Phasentrennung erhaltenen organischen Phasen wurden jeweils wie in Beispiel 1 beschrieben analysiert.

Nachstehende Tabelle enthält eine vergleichende Übersicht der Resultate:

**Tabelle 1: Vergleich der Ergebnisse aus den Beispielen**

| | | | *Erste Basebehandlung, Wasserzugabe und Phasentrennung Schritte (i)* - *(iii)* | | | | *Zweite Basebehandlung, Wasserzugabe und Phasentrennung Schritte (iv) - (vi)* | | | | *Wäsche und Phasentrennung* |
|---|---|---|---|---|---|---|---|---|---|---|---|
| Bsp. Nr. | Gehalt an Phenol im rohen Anilin [ppm] | Molares Verhältnis *in Summe* eingesetzter Base zu Phenol im rohen Anilin [a] | Massenverhältnis von organischem zu wässrigem Anteil im Gemisch aus Schritt (i)^{[b]} | Massenverhältnis des Gemisches aus Schritt (i) zur Menge an zugegebenem Wasser | Gehalt an Phenol in der organischen Phase aus Schritt (iii) [ppm] | Gehalt an Natrium in der organischen Phase aus Schritt (iii) [ppm] | Massenverhältnis von organischem zu wässrigem Anteil im Gemisch aus Schritt (iv) ^{[b]} | Massenverhältnis des Gemisches aus Schritt (iv) zur Menge an zugegebenem Wasser | Gehalt an Phenol in der organischen Phase aus Schritt (vi) [ppm] | Gehalt an Natrium in der organischen Phase aus Schritt (vi) [ppm] | Gehalt an Natrium nach Wäsche und Phasentrennung [ppm] |
| 1 | 998 | 7,53 | 154 | 4,02 | 150 | 52 | 18,7 | 4,02 | 37 | 95 | < 1 |
| 2 | 998 | 7,53 | 154 | 1,01 | 178 | < 1 | 18,7 | 1,01 | 29 | < 1 | < 1 |

| | | | | | | | | | | | |
|---|---|---|---|---|---|---|---|---|---|---|---|
| Erläuterungen: [a]: Berechnet für vollständige Weiterverarbeitung der nach einer Phasentrennung erhaltenen organischen Phase im nächsten Schritt. [b] Unter Berücksichtigung des in der organischen Phase vor Basezugabe bereits vorhandenen Wassergehalts. | | | | | | | | | | | |

Die Beispiele zeigen, dass der sehr hohe Ausgangs-Phenolgehalt von nahezu 1000 ppm mit nur geringen molaren Überschüssen an Base um ca. 97 % gesenkt werden kann. Auf die abschließende Wäsche kann verzichtet werden, wenn die Menge an Wasser, mit der das Gemisch aus Schritt b) (i) bzw. das Gemisch aus Schritt b) (iv) behandelt wird, hinreichend groß ist (Beispiel 2: Natriumgehalt in der organischen Phase auch ohne abschließende Wäsche bereits < 1 ppm).

## Patentansprüche

1. Verfahren zur Herstellung von aromatischen Aminen, bei dem
a) die aromatischen Amine durch Hydrierung der entsprechenden aromatischen Nitroverbindungen in Gegenwart eines Katalysators hergestellt werden und das bei der Hydrierung gebildete Wasser durch Phasentrennung abgetrennt wird, wobei die rohen aromatischen Amine erhalten werden, und
b) die aus Schritt a) erhaltenen rohen aromatischen Amine anschließend gereinigt werden, indem
b) (i) die aus Schritt a) erhaltenen rohen aromatischen Amine mit mindestens einer wässrigen Lösung einer Base vermischt werden, wobei die eingesetzte Menge und die Basen-Konzentration der wässrigen Lösung der Base so gewählt werden, dass das molare Verhältnis der Summe aller Basen, die in Verfahrensschritt b) zu den aromatischen Aminen zugegeben werden, zu den phenolischen Hydroxygruppen, die in den aus Schritt a) erhaltenen aromatischen Aminen enthalten sind, nicht größer als 10 ist, und dass das Massenverhältnis von organischen Bestandteilen zu Wasser größer als 10 ist, und
b) (ii) das aus Schritt b) (i) erhaltene Gemisch mit Wasser vermischt wird, wobei das Massenverhältnis des aus Schritt b) (i) erhaltenen Gemisches zu dem in Schritt b) (ii) zugegebenen Wasser im Bereich zwischen 0,05 und 20 liegt, und
b) (iii) das aus Schritt b) (ii) erhaltene zweiphasige Gemisch anschließend in eine die aromatischen Amine enthaltende organische Phase und eine wässrige Phase aufgetrennt wird.

2. Verfahren nach Anspruch 1, bei dem zusätzlich
b) (iv) die aus Schritt b) (iii) erhaltene die aromatischen Amine enthaltende organische Phase mit mindestens einer wässrigen Lösung einer Base vermischt wird, wobei die eingesetzte Menge und die Basen-Konzentration der wässrigen Lösung der Base so gewählt werden, dass das molare Verhältnis der Summe aller Basen, die in Verfahrensschritt b) zu den aromatischen Aminen zugegeben werden, zu den phenolischen Hydroxygruppen, die in den aus Schritt a) erhaltenen aromatischen Aminen enthalten sind, nicht größer als 10 ist, und dass das Massenverhältnis von organischen Bestandteilen zu Wasser größer als 10 ist, und
b) (v) das aus Schritt b) (iv) erhaltene Gemisch mit Wasser vermischt wird, wobei das Massenverhältnis des aus Schritt b) (iv) erhaltenen Gemisches zu dem in Schritt b) (v) zugegebenen Wasser im Bereich zwischen 0,05 und 20 liegt, und
b) (vi) das aus Schritt b) (v) erhaltene zweiphasige Gemisch anschließend in eine die aromatischen Amine enthaltende organische Phase und eine wässrige Phase aufgetrennt wird.

3. Verfahren nach Anspruch 1 oder 2, bei dem die aus Schritt b) (iii) oder Schritt b) (vi) erhaltene die aromatischen Amine enthaltende organische Phase mit Wasser in einem ein- oder mehrstufigen Prozess gewaschen wird.

4. Verfahren nach Anspruch 1 oder 2, bei dem die Schritte b) (i) - (iii) und ggf. die zusätzlichen Schritte b) (iv) - (vi) bei Temperaturen zwischen 20 °C und 160 °C ausgeführt werden.

5. Verfahren nach einem der Ansprüche 1 oder 2, bei dem das aromatische Amin Anilin ist.

6. Verfahren nach einem der Ansprüche 1 oder 2, bei dem das in Schritt b) (ii) oder Schritt b) (v) zugesetzte Wasser rezykliertes Prozesswasser ist, das aus der Umsetzung in Schritt a) erhalten worden ist.

7. Verfahren nach einem der Ansprüche 1 oder 2, bei dem die eingesetzte Base ein Alkalimetallhydroxid ist.

8. Verfahren nach einem der Ansprüche 1 oder 2, bei dem das aus Schritt a) erhaltene rohe aromatische Amin einen Wassergehalt von 0,01 bis 20 Massen-% , bezogen auf die Masse des aus Schritt a) erhaltenen rohen aromatischen Amins, aufweist.

9. Verfahren nach einem der Ansprüche 1 oder 2, bei dem die nach der in Schritt b) (iii) oder in Schritt b) (vi) oder ggf. einer oder mehreren weiteren Phasentrennungen erhaltene die aromatischen Amine enthaltende organische Phase durch Destillation weiter aufgereinigt wird.

10. Verfahren nach Anspruch 8, bei dem das aus Schritt a) erhaltene rohe aromatische Amin zunächst destillativ gereinigt und anschließend das gereinigte aromatische Amin in Schritt b) weiter aufgereinigt wird.

## Claims

1. Process for the preparation of aromatic amines wherein
a) the aromatic amines are prepared by hydrogenation of the corresponding aromatic nitro compounds in the presence of a catalyst, and the water formed in the hydrogenation is separated off by phase separation to give the crude aromatic amines, and
b) the crude aromatic amines obtained from step a) are then purified by a procedure in which
b) (i) the crude aromatic amines obtained from step a) are mixed with at least one aqueous solution of a base, the amount used and the base concentration of the aqueous base solution being chosen so that the molar ratio of the sum of all the bases added to the aromatic amines in process step b) to the phenolic hydroxyl groups contained in the aromatic amines obtained from step a) is not greater than 10, and so that the weight ratio of organic constituents to water is greater than 10,
b) (ii) the mixture obtained from step b) (i) is mixed with water, the weight ratio of the mixture obtained from step b) (i) to the water added in step b) (ii) being in the range between 0.05 and 20, and b) (iii) the two-phase mixture obtained from step b) (ii) is then separated into an organic phase containing the aromatic amines and an aqueous phase.

2. Process according to Claim 1 wherein additionally
b) (iv) the organic phase obtained from step b) (iii), containing the aromatic amines, is mixed with at least one aqueous solution of a base, the amount used and the base concentration of the aqueous base solution being chosen so that the molar ratio of the sum of all the bases added to the aromatic amines in process step b) to the phenolic hydroxyl groups contained in the aromatic amines obtained from step a) is not greater than 10, and so that the weight ratio of organic constituents to water is greater than 10,
b) (v) the mixture obtained from step b) (iv) is mixed with water, the weight ratio of the mixture obtained from step b) (iv) to the water added in step b) (v) being in the range between 0.05 and 20, and
b) (vi) the two-phase mixture obtained from step b) (v) is then separated into an organic phase containing the aromatic amines and an aqueous phase.

3. Process according to Claim 1 or 2 wherein the organic phase obtained from step b) (iii) or step b) (vi), containing the aromatic amines, is washed with water in a one-stage or multistage process.

4. Process according to Claim 1 or 2 wherein steps b) (i) - (iii) and optionally the additional steps b) (iv) - (vi) are carried out at temperatures between 20°C and 160°C.

5. Process according to Claim 1 or 2 wherein the aromatic amine is aniline.

6. Process according to Claim 1 or 2 wherein the water added in step b) (ii) or step b) (v) is recycled process water obtained from the reaction in step a).

7. Process according to Claim 1 or 2 wherein the base used is an alkali metal hydroxide.

8. Process according to Claim 1 or 2 wherein the crude aromatic amine obtained from step a) has a water content of 0.01 to 20 wt.%, based on the weight of the crude aromatic amine obtained from step a).

9. Process according to Claim 1 or 2 wherein the organic phase obtained after the phase separation in step b) (iii) or step b) (vi), or optionally one or more additional phase separations, containing the aromatic amines, is purified further by distillation.

10. Process according to Claim 8 wherein firstly the crude aromatic amine obtained from step a) is purified by distillation and then the purified aromatic amine is purified further in step b).

## Revendications

1. Procédé pour la préparation d'amines aromatiques, dans lequel
a) les amines aromatiques sont préparées par hydrogénation des composés nitro aromatiques correspondants en présence d'un catalyseur et l'eau formée lors de l'hydrogénation est séparée par séparation des phases, les amines aromatiques brutes étant obtenues, et
b) les amines aromatiques brutes obtenues à partir de l'étape a) sont ensuite purifiées en ce que
b)(i) les amines aromatiques brutes obtenues à partir de l'étape a) sont mélangées avec au moins une solution aqueuse d'une base, la quantité utilisée et la concentration en base de la solution aqueuse de base étant choisies de manière telle que le rapport molaire de la somme de toutes les bases, qui sont ajoutées dans l'étape de procédé b) aux amines aromatiques, aux groupes hydroxy phénoliques, qui sont contenus dans les amines aromatiques obtenues dans l'étape a), n'est pas supérieur à 10 et que le rapport massique de constituants organiques à eau est supérieur à 10 et
b)(ii) le mélange obtenu de l'étape b)(i) est mélangé avec de l'eau, le rapport massique du mélange obtenu de l'étape b)(i) à l'eau ajoutée dans l'étape b)(ii) étant situé dans la plage entre 0,05 et 20 et
b)(iii) le mélange à deux phases obtenu de l'étape b)(ii) est ensuite séparé en une phase organique contenant les amines aromatiques et une phase aqueuse.

2. Procédé selon la revendication 1, dans lequel, en plus,
b)(iv) la phase organique contenant les amines aromatiques, obtenue à partir de l'étape b)(iii) est mélangée avec au moins une solution aqueuse d'une base, la quantité utilisée et la concentration en base de la solution aqueuse de base étant choisies de manière telle que le rapport molaire de la somme de toutes les bases, qui sont ajoutées dans l'étape de procédé b) aux amines aromatiques, aux groupes hydroxy phénoliques, qui sont contenus dans les amines aromatiques obtenues dans l'étape a), n'est pas supérieur à 10 et que le rapport massique de constituants organiques à eau est supérieur à 10 et
b)(v) le mélange obtenu de l'étape b)(iv) est mélangé avec de l'eau, le rapport massique du mélange obtenu de l'étape b)(iv) à l'eau ajoutée dans l'étape b)(v) étant situé dans la plage entre 0,05 et 20 et
b)(vi) le mélange à deux phases obtenu de l'étape b)(v) est ensuite séparé en une phase organique contenant les amines aromatiques et une phase aqueuse.

3. Procédé selon la revendication 1 ou 2, dans lequel la phase organique contenant les amines aromatiques obtenue de l'étape b)(iii) ou de l'étape b)(vi) est lavée avec de l'eau dans un procédé à une ou plusieurs étapes.

4. Procédé selon la revendication 1 ou 2, dans lequel les étapes b)(i)-(iii) et le cas échéant les étapes supplémentaires b)(iv)-(vi) sont réalisées à des températures entre 20°C et 160°C.

5. Procédé selon l'une quelconque des revendications 1 ou 2, dans lequel l'amine aromatique est l'aniline.

6. Procédé selon l'une quelconque des revendications 1 ou 2, dans lequel l'eau ajoutée dans l'étape b)(ii) ou b)(v) est de l'eau de procédé recyclée, qui a été obtenue à partir la transformation dans l'étape a).

7. Procédé selon l'une quelconque des revendications 1 ou 2, dans lequel la base utilisée est un hydroxyde de métal alcalin.

8. Procédé selon l'une quelconque des revendications 1 ou 2, dans lequel l'amine aromatique brute obtenue de l'étape a) présente une teneur en eau de 0,01 à 20% en masse, par rapport à la masse de l'amine aromatique brute obtenue de l'étape a).

9. Procédé selon l'une quelconque des revendications 1 ou 2, dans lequel la phase organique contenant des amines aromatiques, obtenue après la séparation des phases dans l'étape b)(iii) ou dans l'étape b)(vi) ou le cas échéant après une ou plusieurs autres séparations des phases est purifiée davantage par distillation.

10. Procédé selon la revendication 8, dans lequel l'amine aromatique brute obtenue de l'étape a) est d'abord purifiée par distillation et l'amine aromatique purifiée est ensuite purifiée davantage dans l'étape b).
